(19) Europäisches Patentamt
European Patent Office
Office européen des brevets

(11) **EP 2 203 113 B1**

(12) **EUROPÄISCHE PATENTSCHRIFT**

(45) Veröffentlichungstag und Bekanntmachung des Hinweises auf die Patenterteilung:
**25.02.2015 Patentblatt 2015/09**

(21) Anmeldenummer: **08801909.6**

(22) Anmeldetag: **08.09.2008**

(51) Int Cl.:
*A61B 5/00* (2006.01)          *A61B 5/01* (2006.01)
*A61B 5/02* (2006.01)          *A61B 5/022* (2006.01)
*A61B 5/024* (2006.01)         *A61B 5/0402* (2006.01)
*A61B 5/053* (2006.01)         *A61B 5/1455* (2006.01)

(86) Internationale Anmeldenummer:
**PCT/EP2008/007330**

(87) Internationale Veröffentlichungsnummer:
**WO 2009/033624 (19.03.2009 Gazette 2009/12)**

(54) **DIAGNOSTISCHE SENSOREINHEIT**

DIAGNOSTIC SENSOR UNIT

ENSEMBLE CAPTEUR DE DIAGNOSTIC

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **07.09.2007 DE 102007042551**

(43) Veröffentlichungstag der Anmeldung:
**07.07.2010 Patentblatt 2010/27**

(73) Patentinhaber: **Flore, Ingo**
**44141 Dortmund (DE)**

(72) Erfinder:
• **CHO, Ok Kyung**
**58239 Schwerte (DE)**

• **KIM, Yoon Ok**
**58239 Schwerte (DE)**

(74) Vertreter: **Isfort, Olaf**
**Schneiders & Behrendt**
**Rechts- und Patentanwälte**
**Huestraße 23**
**44787 Bochum (DE)**

(56) Entgegenhaltungen:
WO-A-2006/099988          WO-A-2007/017266
WO-A1-2008/061788         US-A1- 2003 036 685

## Beschreibung

Die Erfindung betrifft eine diagnostische Sensoreinheit zur nicht-invasiven Erfassung von wenigstens einem physiologischen Parameter von hautoberflächennahem Körpergewebe,

- mit einer optischen Messeinheit , die zwei oder mehr Strahlungsquellen zur Bestrahlung des zu untersuchenden Körpergewebes und wenigstens einen Strahlungssensor zur Detektion der von dem Körpergewebe gestreuten und/oder transmittierten Strahlung umfasst,

- mit einer EKG-Einheit zur Erfassung eines EKG-Signals über zwei oder mehr EKG-Elektroden ,

- mit einer Temperatur- oder Wärmemesseinheit , die mit einem Temperatur- oder Wärmesensor verbunden ist,

- mit einer bioelektrischen Impedanzmesseinheit, und

- mit einem Sensorgehäuse,
  wobei

- die zwei oder mehr Strahlungsquellen , der wenigstens eine Strahlungssensor, die EKG-Einheit, die Temperatur- oder Wärmemesseinheit und die bioelektrische Impedanzmesseinheit in dem Sensorgehäuse angeordnet sind,

- an der Gehäuseoberfläche des Sensorgehäuses wenigstens eine EKG-Elektrode der EKG-Einheit angeordnet ist, in der Weise, dass die EKG-Elektrode die Hautoberfläche in dem von der optischen Messeinheit erfassten Bereich des Körpergewebes berührt, und

- an der Gehäuseoberfläche des Sensorgehäuses wenigstens eine Einspeise- oder Messelektrode der Impedanzmesseinheit angeordnet ist.

Ähnliche diagnostische Sensoreinheiten sind beispielsweise aus WO 2006/099988 A1 und WO 2007/017266 A2 bekannt.

Die Versorgung des Körpergewebes mit Sauerstoff gehört bekanntlich zu den wichtigsten Vitalfunktionen des Menschen. Aus diesem Grund sind oximetrische Diagnosemodalitäten heutzutage von großer Bedeutung in der Medizin. Routinemäßig werden so genannte Pulsoximeter eingesetzt. Die diagnostische Sensoreinheit derartiger Pulsoximeter umfasst typischerweise eine optische Messeinheit mit zwei Lichtquellen, die rotes bzw. infrarotes Licht unterschiedlicher Wellenlänge in das Körpergewebe einstrahlen. Das Licht wird im Körpergewebe gestreut und teilweise absorbiert. Das gestreute Licht wird schließlich mittels eines Lichtsensors in Form einer geeigneten Photozelle (Photodiode) detektiert. Typischerweise verwenden kommerzielle Pulsoximeter zum einen Licht im Wellenlängenbereich von 660 nm. In diesem Bereich ist die Lichtabsorption von Oxihämoglobin und Desoxihämoglobin stark unterschiedlich. Dementsprechend variiert die Intensität des mittels des Fotosensors detektierten, gestreuten Lichts in Abhängigkeit davon, wie stark das untersuchte Körpergewebe von sauerstoffreichem, bzw. sauerstoffarmem Blut durchblutet ist. Zum anderen wird üblicherweise Licht im Wellenlängenbereich von 810 nm verwendet. Diese Lichtwellenlänge liegt im so genannten nahen infraroten Spektralbereich. Die Lichtabsorption von Oxihämoglobin und Desoxihämoglobin ist in diesem Spektralbereich im Wesentlichen gleich. Die bekannten Pulsoximeter sind außerdem in der Lage, ein plethysmographisches Signal, d. h. ein Volumenpulssignal zu erzeugen, das die während des Herzschlags veränderliche Blutmenge in dem von dem Pulsoximeter erfassten Mikrogefäßsystem wiedergibt (sog. Photoplethysmographie). Bei Verwendung unterschiedlicher Lichtwellenlängen in den oben erwähnten Spektralbereichen kann aus der unterschiedlichen Lichtabsorption auf den Sauerstoffgehalt des Blutes (Sauerstoffsättigung) zurückgeschlossen werden. Die üblichen Pulsoximeter werden entweder an der Fingerspitze eines Patienten oder auch am Ohrläppchen eingesetzt. Es wird dann das Volumenpulssignal aus der Blutperfusion des Mikrogefäßsystems in diesen Bereichen des Körpergewebes erzeugt.

Das EKG (Elektrokardiogramm) dürfte die am meisten eingesetzte Untersuchungsmodalität zur Diagnose von Herz-Kreislauf-Erkrankungen sein. Mittels der diagnostischen Sensoreinheit eines EKG-Gerätes werden mit zwei oder mehr EKG-Elektroden elektrische Signale von dem Körper des zu untersuchenden Patienten abgeleitet. Das so gewonnene EKG gibt die bioelektrischen Spannungen, die bei der Erregungsausbreitung und -rückbildung am Herzen entstehen, wieder. Das EKG enthält zahlreiche diagnostisch auswertbare Parameter. Zum Zeitpunkt der Kontraktion des Herzmuskels während eines Herzschlags zeigt das EKG eine deutliche Spitze, die auch als R-Zacke bezeichnet wird. Weiterhin enthält das EKG die der R-Zacke vorangehende, so genannte P-Welle. Der R-Zacke folgt wiederum die so genannte T-Welle. Die Minima im EKG unmittelbar vor und unmittelbar nach der R-Zacke werden mit Q bzw. S bezeichnet. Für die Herz-Kreislauf-Diagnostik interessante Parameter sind die Dauer der P-Welle sowie die Amplitude der P-Welle, die Dauer des PQ-Intervalls, die Dauer des QRS-Komplexes, die Dauer des QT-Intervalls sowie die Amplitude der T-Welle. Sowohl aus den Absolutwerten der genannten Parameter wie auch aus den Verhältnissen der Parameter kann auf den Gesundheitszustand des Herz-Kreislauf-Systems geschlossen werden.

Es ist in jüngerer Zeit bekannt geworden, dass die kombinierte Anwendung verschiedener Diagnosemodalitäten, beispielsweise der Pulsoximetrie mit der EKG-Messung, besonders vorteilhaft ist, um, z.B. im Rahmen von Gesundheits-Screenings, schnell und zuverlässig Informationen über den Gesundheitszustand des Patienten hinsichtlich etwaiger Erkrankungen des Herz-/Kreislaufsystems und etwaiger Stoffwechselerkrankungen zu erhalten.

Der vorliegenden Erfindung liegt vor diesem Hintergrund die Aufgabe zugrunde, eine diagnostische Sensoreinheit zur nicht-invasiven Bestimmung von physiologischen Parametern bereit zu stellen, die gegenüber dem Stand der Technik hinsichtlich ihrer Funktionalität erweitert ist, die in ein beliebiges Accessoire, wie z.B. eine Brille, eine Armbanduhr, ein Schmuckstück oder dergleichen, oder in ein Kleidungsstück (sog. "Smart Clothes") integriert werden kann und die flexibel und kostengünstig in unterschiedliche Gehäuse verschiedener am Markt vorhandener Geräte eingebaut werden kann. Insbesondere soll eine Sensoreinheit geschaffen werden, die einerseits kostengünstig herstellbar ist und andererseits vom Benutzer komfortabel und einfach verwendet werden kann, um eine zuverlässige und frühzeitige Erkennung von Erkrankungen, z.B. auch im Wege der Selbstdiagnose, sowie eine kontinuierliche Überwachung bestehender Erkrankungen zu ermöglichen.

Diese Aufgabe löst die Erfindung ausgehend von einer Sensoreinheit der eingangs angegebenen Art dadurch, dass die wenigstens eine EKG-Elektrode flächig und als Blech aus elektrisch leitendem Material ausgebildet ist, und die EKG-Elektrode wenigstens eine Ausnehmung für den Durchtritt der von den zwei oder mehr Strahlungsquellen emittierten Strahlung in das zu untersuchende Körpergewebe, eine Ausnehmung für den Durchtritt des im Körpergewebe gestreuten Lichtes und eine weitere Ausnehmung für den Temperatur- oder Wärmesensor aufweist, dass die zwei oder mehr Strahlungsquellen mit einem lichtleitenden Element verbunden sind, welches die Strahlung der wenigstens zwei Strahlungsquellen zur Oberfläche des Sensorgehäuses leitet, und die zwei oder mehr Strahlungsquellen, der wenigstens eine Strahlungssensor, die EKG-Einheit, der Temperatur- oder Wärmesensor und die bioelektrische Impedanzmesseinheit auf einer gemeinsamen Platine innerhalb des Sensorgehäuses angeordnet sind.

Durch die erfindungsgemäße Integration einer optischen Messeinheit und einer EKG-Einheit wird eine kompakte Sensoreinheit geschaffen, die eine Vielzahl von diagnostischen Messwerten liefert. Diese können einzeln oder in Kombination ausgewertet werden, um schnell und zuverlässig aussagekräftige Informationen zum Gesundheitszustand des untersuchten Patienten zu erhalten. Die kompakte Sensoreinheit kann als vollständig funktionsfähiges Teil kostengünstig in großen Stückzahlen vorgefertigt und in Diagnosegeräte verschiedenster Art integriert werden. Die eigentliche Messung kann besonders einfach und komfortabel durchgeführt werden. Hierzu wird die Oberfläche des Sensorgehäuses mit der Haut im Bereich des zu untersuchenden Körpergewebes in Kontakt gebracht, was z.B. durch Auflegen eines Fingers des Patienten auf die Gehäuseoberfläche der Sensoreinheit erfolgen kann. Die optische Messung und die EKG-Ableitung erfolgen dann gleichzeitig über die die Sensoreinheit berührende Hautstelle.

Die diagnostische Sensoreinheit umfasst gemäß der Erfindung eine optische Messeinheit zur Erzeugung von oximetrischen und/oder plethysmographischen Messsignalen. Dies ermöglicht es, die Versorgung des Körpergewebes des Benutzers der Vorrichtung mit Sauerstoff zu überwachen und/oder ein Volumenpulssignal zu erzeugen.

Die optische Messeinheit der erfindungsgemäßen Sensoreinheit weist zwei oder mehr Strahlungsquellen zur Bestrahlung des untersuchten Körpergewebes mit elektromagnetischer Strahlung, und wenigstens einen Strahlungssensor zur Detektion der von dem Körpergewebe gestreuten und/oder transmittierten Strahlung auf. Als Strahlungsquellen kommen übliche Leuchtdioden oder auch Laserdioden in Frage, die optische Strahlung, d.h. Licht im entsprechenden Spektralbereich emittieren. Als besonders vorteilhaft hat es sich erwiesen, wenn mit der erfindungsgemäßen Vorrichtung die Strahlungsabsorption im untersuchten Körpergewebe bei mindestens zwei oder besser drei unterschiedlichen Lichtwellenlängen gemessen wird, um daraus die Sauerstoffkonzentration des Blutes und die Durchblutung des Gewebes zu bestimmen.

Gemäß einer sinnvollen Ausgestaltung weist die optische Messeinheit der erfindungsgemäßen Sensoreinheit wenigstens zwei Strahlungssensoren zur Detektion der von dem Körpergewebe gestreuten und/oder transmittierten Strahlung auf, wobei die Strahlungssensoren in unterschiedlichem Abstand zur Strahlungsquelle angeordnet sind. Dies eröffnet die Möglichkeit, Rückschlüsse auf die jeweils im Körpergewebe von der Strahlung zurückgelegte Strecke zu ziehen. Auf dieser Basis kann die Sauerstoffkonzentration im Blut und im Gewebe in unterschiedlich tiefen Gewebeschichten untersucht werden. Dabei kann ausgenutzt werden, dass die Messsignale aus den tiefer liegenden Gewebeschichten stärker vom arteriellen Blut beeinflusst sind, während in den oberflächennäheren Regionen die Strahlungsabsorption stärker von dem Blut im kapillaren Gefäßsystem beeinflusst ist.

Vorteilhaft ist eine Ausgestaltung der erfindungsgemäßen Sensoreinheit, bei welcher wenigstens zwei Strahlungsquellen vorgesehen sind, welche unterschiedliche Volumenbereiche des untersuchten Körpergewebes bestrahlen. Hierdurch lässt sich eine differenzielle Messung der Lichtabsorption einfach realisieren. Dies ermöglicht es, Metabolismus-induzierte Änderungen der Durchblutung des untersuchten Körpergewebes mit sauerstoffreichem bzw. sauerstoffarmem Blut zu untersuchen. Dabei wird ausgenutzt, dass sich in Abhängigkeit von der metabolischen Aktivität des Gewebes der lokale Sauerstoffverbrauch verändert. Die Bestimmung des veränderlichen Sauerstoffverbrauchs erlaubt wiederum Rückschlüsse auf den lokalen Energieverbrauch, der mit dem Sauerstoffverbrauch direkt korreliert ist. Besonders interessant ist, dass dies wiederum Rückschlüsse auf den Glukosespiegel zulässt. Somit erlaubt die erfindungsgemäße Sensoreinheit vorteilhafterweise auch eine nicht-invasive Bestimmung des Blutglukosespiegels.

Die wenigstens zwei Strahlungsquellen der optischen Messeinheit der erfindungsgemäßen Sensoreinheit sollten so ausgelegt sein, dass die von diesen jeweils bestrahlten Volumenbereiche hinsichtlich der Durchblutung mit sauerstoffarmem bzw. sauerstoffreichem Blut unterschiedlich betroffen sind. Dies kann z. B. dadurch erreicht werden, dass die wenigstens zwei Strahlungsquellen unterschiedliche räumliche Abstrahlcharakteristiken haben. So können als Strahlungsquellen z. B. eine Leuchtdiode und ein Laser verwendet werden, die ähnliche Wellenlängen (z. B. 630 nm und 650 nm) haben. Die beiden Strahlungsquellen unterscheiden sich aber durch den Öffnungswinkel der Abstrahlung. Während z. B. die Leuchtdiode unter einem großen Öffnungswinkel in das untersuchte Körpergewebe einstrahlt, tritt das Licht der Laserdiode unter einem sehr kleinen Öffnungswinkel in das Körpergewebe ein. Dies hat zur Folge, dass mit den beiden Strahlungsquellen unterschiedliche Volumenbereiche des Körpergewebes erfasst werden. Aufgrund des großen Öffnungswinkels wird von der Leuchtdiode ein größerer Volumenbereich der nicht-durchbluteten Epidermis erfasst als von dem Laser. Die undurchblutete Epidermis ist von einer Änderung der Hämoglobinkonzentration praktisch nicht betroffen. Dementsprechend ist die Intensität der von dem Körpergewebe gestreuten und/oder transmittierten Strahlung der Leuchtdiode weniger stark von einer Änderung der Hämoglobinkonzentration abhängig als die Intensität der Strahlung des Lasers. Voraussetzung ist, dass die Wellenlänge der von den beiden Strahlungsquellen jeweils emittierten Strahlung so gewählt wird, dass die Strahlung unterschiedlich stark durch Oxihämoglobin bzw. Desoxihämoglobin absorbiert wird. Die Wellenlänge sollte daher zwischen 600 und 700 nm, vorzugsweise zwischen 630 und 650 nm liegen.

Gemäß einer sinnvollen Ausgestaltung der Sensoreinheit sind die zwei oder mehr Strahlunqsquellen mit einem lichtleitenden Element, z.B. einer optischen Faser verbunden. Über das lichtleitende Element wird die von den Strahlungsquellen emittierte Strahlung zur Oberfläche des Sensorgehäuses geleitet. Es besteht die vorteilhafte Möglichkeit, die Strahlung mehrer Strahlungsquellen, z.B. mehrerer LED-Chips, die auf ein gemeinsames Substrat gebondet sind, in ein einziges lichtleitendes Element einzukoppeln. Dabei können die verschiedenen Strahlungsquellen in unterschiedlicher Weise an das lichtleitende Element gekoppelt sein. Auf diese Weise lässt sich eine unterschiedliche Abstrahlcharakteristik der Strahlung der verschiedenen Quellen in das zu untersuchende Körpergewebe erzielen.

Die erfindungsgemäße Sensoreinheit kann mit Vorteil zur Bestimmung eines lokalen metabolischen Parameters aus der von dem Körpergewebe gestreuten und/oder transmittierten Strahlung der wenigsten zwei Strahlungsquellen ausgebildet sein. Wenn in dem untersuchten Körpergewebe Sauerstoff verbraucht wird, wird Oxihämoglobin in Desoxihämoglobin umgewandelt. Durch einen Vergleich der aus den unterschiedlichen Volumenbereichen des Körpergewebes stammenden Strahlung der beiden Strahlungsquellen kann die Änderung des Konzentrationsverhältnisses von Oxihämoglobin und Desoxihämoglobin festgestellt werden. Hieraus ergibt sich wiederum der lokale Sauerstoffverbrauch und daraus letztlich (indirekt) der Blutglukosespiegel.

Die EKG-Einheit der erfindungsgemäßen Sensoreinheit dient zur Erfassung eines EKG-Signals über zwei oder mehr EKG-Elektroden. Hierdurch wird der Funktionsumfang der erfindungsgemäßen Sensoreinheit gegenüber herkömmlichen Systemen vorteilhaft erweitert. Die erfindungsgemäße Sensoreinheit ermöglicht es, pulsoximetrische Signale und EKG-Signale kombiniert zu erfassen und auszuwerten. Zweckmäßigerweise ist hierzu eine Auswertungseinheit zur Auswertung des zeitlichen Verlaufs der optisch gemessenen Volumenpulssignale und der EKG-Signale vorgesehen. Diese Auswertungseinheit kann integraler Bestandteil der Sensoreinheit sein. Ebenso kann vorgesehen sein, dass die Auswertungseinheit von der Sensoreinheit separat ist, wobei die Messsignale über eine geeignete Datenverbindung von der Sensoreinheit an die Auswertungseinheit übertragen werden. Mittels einer geeigneten Programmsteuerung ist die Auswertungseinheit dazu in der Lage, die R-Zacken in dem EKG-Signal automatisch zu erkennen. Damit wird automatisch der exakte Zeitpunkt des Herzschlags ermittelt. Weiterhin ist die Auswertungseinheit aufgrund geeigneter Programmsteuerung dazu in der Lage, die Maxima in dem Volumenpulssignal zu erkennen. Anhand der Maxima in dem Volumenpulssignal ist der Zeitpunkt des Eintreffens einer bei einem Herzschlag ausgelösten Pulswelle an dem von der Sensoreinheit erfassten peripheren Messort feststellbar. Somit kann schließlich der zeitliche Abstand zwischen einer R-Zacke in dem EKG-Signal und dem darauf folgenden Maximum in dem Volumenpulssignal ermittelt werden. Dieser zeitliche Abstand ist ein Maß für die so genannte Pulswellengeschwindigkeit. Auf der Basis der Pulswellengeschwindigkeit kann einerseits eine Aussage über den Blutdruck getroffen werden. Eine Verkürzung der Pulswellengeschwindigkeit geht nämlich mit einer Erhöhung des Blutdrucks einher, während eine Verlängerung der Pulswellengeschwindigkeit auf eine Blutdruckerniedrigung schließen lässt. Eine exakte Bestimmung des Blutdrucks aus der Pulswellengeschwindigkeit ist allerdings nicht möglich, es können nur Tendenzen angegeben werden. Weiterhin ist die Pulswellengeschwindigkeit von der Dichte des Blutes und insbesondere von der Elastizität der Blutgefäßwandungen (beispielsweise der Aorta) abhängig. Aus der Elastizität der Blutgefäße kann wiederum auf eine ggf. vorliegende Arteriosklerose geschlossen werden. Es können in diese Auswertung auch die Absolutwerte der Herzfrequenz, die Herzfrequenzvariabilität und entsprechende Arrhythmien des Herzens einbezogen werden. So können automatisch Arrhythmien wie Sinus Tachycardia, Sinus Bradycardia, Sinus Arrest und so genannte Escape Beats festgestellt werden. Anhand des EKG-Signals können außerdem Aussagen über die zeitliche Dauer der Vorhofkontraktion des Herzens bei einem Herzschlag, die zeitliche Dauer der Herzkammerkontraktion sowie die Dauer der Relaxation der Herzkammer usw. festgestellt werden. Außerdem sind Vordiagnosen bezüglich so genannter Blocks in der Leitung der elektrischen Erregungssignale am Herzen (AV-Block, Bundle Branch-Block usw.) und auch bezüglich Durchblutungsstörungen oder Infarkten möglich. Weitere Irregularitäten im Pulsverlauf sind anhand des Volumenpulssignals feststellbar.

Eine der wenigstens zwei EKG-Elektroden ist gemäß der Erfindung an der Oberfläche des Sensorgehäuses angeordnet, welches auch die übrigen Messeinheiten enthält. Die andere EKG-Elektrode ist zweckmäßigerweise so angeordnet, dass der Patient beide Elektroden mit verschiedenen Extremitäten berühren kann, beispielsweise mit jeder Hand jeweils eine der Elektroden.

Die Erfindung basiert u. a. auf der Erkenntnis, dass durch die Kombination verschiedener Diagnosemodalitäten in einer einzigen Sensoreinheit die Möglichkeit eröffnet wird, lokale metabolische Parameter zu bestimmen.

Beispielsweise für die Ermittlung des lokalen Sauerstoffverbrauchs kann mittels der erfindungsgemäßen Sensoreinheit zusätzlich zur oximetrisch bestimmten arteriellen Sauerstoffkonzentration auch die kapillare Sauerstoffkonzentration im Gewebe bestimmt werden können. Hierzu muss allerdings die Zusammensetzung des untersuchten Körpergewebes bekannt sein. Entscheidende Parameter sind der lokale Fettgehalt und/oder der Wassergehalt des Körpergewebes. Diese Parameter können beispielsweise mittels bioelektrischer Impedanzmessung erfasst werden.

Gemäß der Erfindung ist also eine herkömmliche (optische) Oximetrieeinheit nicht nur mit einer EKG-Einheit, sondern auch mit einer bioelektrischen Impedanzmesseinheit in einer einzigen Sensoreinheit kombiniert. Aus den mittels der bioelektrischen Impedanzmesseinheit gewonnenen Messsignalen kann die Zusammensetzung des untersuchten Körpergewebes bestimmt werden. Auf dieser Grundlage kann dann, z.B. mittels einer geeigneten programmgesteuerten Auswertungseinheit, die mit den Messeinheiten der erfindungsgemäßen Sensoreinheit verbunden ist, aus den oximetrischen Signalen der Sensoreinheit die kapillare Sauerstoffsättigung im Gewebe ermittelt werden. Die arterielle Sauerstoffsättigung ($SaO_2$) und die venöse Sauerstoffsättigung ($SvO_2$) bestimmen abhängig von der Art des untersuchten Gewebes die kapillare (arteriovenöse) Sauerstoffsättigung ($StO_2$). Es gilt:

$$K * SvO_2 + (1 - K) * SaO_2 = StO_2,$$

wobei K ein gewebeabhängiger Korrekturfaktor ist, der vom Volumenverhältnis von Arterien zu Venen im untersuchten Gewebe abhängt. Im Mittel liegt dieser Wert etwas unter 0,5. Der für das jeweilige Gewebe maßgebliche Wert kann gemäß der Erfindung durch bioelektrische Impedanzmessung ermittelt werden, um dann aus der obigen Formel die venöse Sauerstoffsättigung zu bestimmen. Die erfindungsgemäße Sensoreinheit kann genutzt werden, um die Durchblutung V, d. h. die durchblutungsbedingte Volumenschwankung des untersuchten Körpergewebes zu bestimmen. Nach der Beziehung

$$VO_2 = V * (SaO_2 - SvO_2)$$

kann dann schließlich der lokale Sauerstoffverbrauch $VO_2$ berechnet werden, der ein Maß für die metabolische Aktivität am Messort darstellt.

Für die bioelektrische Impedanzmessung sind zweckmäßigerweise Einspeise- oder Messelektroden an der Gehäuseoberfläche des Sensorgehäuses angeordnet, so dass gleichzeitig mit der oximetrischen und der EKG-Messung die Bioimpedanzmessung erfolgen kann. Dabei wird der selbe Bereich des Körpergewebes, nämlich dort, wo der Patient die Oberfläche des Sensorgehäuses berührt, von sämtlichen Messmodalitäten gleichzeitig erfasst.

Die erfindungsgemäße Sensoreinheit umfasst einen integrierten Temperatur- oder Wärmesensor. Dieser kann zur Bestimmung der lokalen Wärmeproduktion genutzt werden. Im einfachsten Fall ist der Temperatursensor (z.B. ein NTC-Element) zur Messung der Oberflächentemperatur der Haut am Messort ausgebildet. Vorzugsweise ist mittels des Wärmesensors eine orts-, zeit- und tiefenaufgelöste Wärmemessung am Messort möglich. Anhand des Wärmeaustauschs kann auf die lokale Stoffwechselaktivität zurückgeschlossen werden. Außerdem ist der Wärmesensor zur Bestimmung der lokalen Durchblutung geeignet. Bezüglich näherer Hintergrundinformationen zur Wärmemessung wird auf die Veröffentlichung von Nitzan et al. verwiesen (Meir Nitzan, Boris Khanokh, "Infrared Radiometry of Thermally Insulated Skin for the Assessment of Skin Blood Flow", Optical Engineering 33, 1994, No. 9, S. 2953 bis 2956). Insgesamt liefert der Wärmesensor Daten, die mit Vorteil zur Bestimmung von metabolischen Parametern genutzt werden können.

Besonders vorteilhaft ist die erfindungsgemäße Kombination der vorgenannten Messverfahren, nämlich der Oximetrie, der EKG-Messung, der Temperatur- bzw. Wärmemessung und der bioelektrischen Impedanzmessung. Mittels der oben erwähnten programmgesteuerten Auswertungseinheit können sämtliche Messsignale durch einen geeigneten Algorithmus ausgewertet und kombiniert werden. Durch die Kombination der verschiedenen Messmodalitäten wird eine hohe Effektivität und Zuverlässigkeit bei der Erkennung von pathologischen Veränderungen erreicht. Sämtliche Parameter können mit Vorteil zu einem globalen Index zusammengefasst werden, der für den Benutzer leicht interpretierbar ist und ihm einen direkten und fundierten Hinweis auf seinen allgemeinen Gesundheitszustand gibt.

Die Kombination der verschiedenen Messmodalitäten, die in der erfindungsgemäßen Sensoreinheit, wie oben beschrieben, zusammengefasst sind, ist weiterhin vorteilhaft, weil dadurch eine nicht-invasive indirekte Messung der Glukosekonzentration möglich ist. Ein mögliches Vorgehen bei der Bestimmung des Blutglukosespiegels mittels der erfindungsgemäßen Vorrichtung wird im Folgenden näher erläutert:

Die erfindungsgemäße Sensoreinheit dient zur Messung von Daten, die durch den Stoffwechsel beeinflusst werden. Es leuchtet unmittelbar ein, dass dabei der Energiehaushaft und die Zusammensetzung der von einem untersuchten Patienten aufgenommenen Nahrung eine große Rolle spielen. Die Nährstoffe, die am Stoffwechsel beteiligt sind, sind bekanntlich im Wesentlichen Kohlenhydrate, Fette und Eiweiße. Kohlenhydrate werden zur weiteren Verarbeitung in Glukose, Eiweiße in Aminosäuren, und Fette in Fettsäuren umgewandelt. Die Energieträger werden dann wiederum in den Zellen des Körpergewebes zusammen mit Sauerstoff unter Abgabe von Energie zu ATP (Adenosintriphosphorsäure) umgewandelt. ATP ist der eigentliche körpereigene Energieträger. Die Verwendung von Glukose zur Erzeugung von ATP ist bevorzugt. Wenn die Erzeugung von ATP aus Glukose jedoch (z. B. wegen eines Mangels an Insulin) gehemmt ist, findet stattdessen eine verstärkte Fettsäure-Oxidation statt. Der Sauerstoffverbrauch ist bei diesem Prozess allerdings ein anderer.

Die Reaktion des Metabolismus des menschlichen Körpers auf eine Nahrungsaufnahme hängt, wie zuvor erwähnt, von der Zusammensetzung der Nahrung charakteristisch ab. So reagiert beispielsweise das vaskuläre System des Körpers in Abhängigkeit davon, wie viel Energie der Körper zur Verdauung der aufgenommenen Speisen benötigt. Anhand der mittels der erfindungsgemäßen Sensoreinheit bestimmbaren Pulswellengeschwindigkeit sowie auch anhand der Blutdruckamplitude und des Pulses lässt sich die Reaktion des Körpers auf die Nahrungsaufnahme bestimmen. Die Pulswellengeschwindigkeit, sowie auch die Blutdruckamplitude und der Puls ändern sich, sobald die Nahrungsaufnahme beginnt. Die Maxima und die jeweiligen Zeitpunkte der Maxima sind dabei beeinflusst durch die Nahrungszusammensetzung. Der Verlauf und die absolute Höhe von Pulswellengeschwindigkeit, Blutdruckamplitude und Puls können herangezogen werden, um die Zusammensetzung der aufgenommenen Nahrung zu bestimmen.

Der Metabolismus des menschlichen Körpers ist im Normalzustand, d. h. in Ruhe und in der so genannten thermoneutralen Zone, im Wesentlichen durch den Glukosehaushalt bestimmt. Daher kann die Glukosekonzentration in den Zellen des Körpergewebes in diesen Normalzustand als reine Funktion der Wärmeproduktion und des Sauerstoffverbrauchs beschrieben werden. Es gilt:

$$[Glu] = f_1(\Delta T, VO_2) \, ,$$

wobei [Glu] für die Glukosekonzentration steht. Die Wärmeproduktion $\Delta T$ kann mittels des Wärmesensors der erfindungsgemäßen Sensoreinheit z.B. aus der Differenz zwischen der arteriellen Temperatur und der Temperatur, welche die Hautoberfläche bei perfekter thermischer Isolierung erreichen würde, bestimmt werden ($\Delta T = T_\infty - T_{Arterie}$). $f_1(\Delta T, VO_2)$ gibt die funktionale Abhängigkeit der Glukosekonzentration von der Wärmeproduktion und vom Sauerstoffverbrauch an. Der Sauerstoffverbrauch ergibt sich, wie oben beschrieben, aus dem Unterschied zwischen venöser und arterieller Sauerstoffsättigung und der Durchblutung. Zur Bestimmung der Glukosekonzentration während bzw. direkt nach der Nahrungsaufnahme muss jedoch ein Korrekturterm berücksichtigt werden, der den Anteil des Fettstoffwechsels am Energiehaushalt wiedergibt. Es gilt dann:

$$[Glu] = f_1(\Delta T, VO_2) + X * f_2(\Delta T, VO_2)$$

X ist ein Faktor, der nach der Nahrungsaufnahme negativ ist. Dabei hängt X von der Zusammensetzung der aufgenommenen Nahrung ab. Insbesondere ist X davon abhängig, in welchem Verhältnis Fett und Kohlenhydrate am Metabolismus beteiligt sind. Der Faktor X lässt sich, wie oben beschrieben, anhand des zeitlichen Verlaufs der Pulswellengeschwindigkeit bestimmen. X ist 0, wenn reine Kohlenhydrate oder direkt Glukose aufgenommen werden. Der Betrag von X steigt an, je größer der Anteil von Fett an der aufgenommenen Nahrung ist. Zur Bestimmung des Korrekturfaktors X aus dem zeitlichen Verlauf der Pulswellengeschwindigkeit, der Blutdruckamplitude und/oder des Pulses wird normalerweise eine Eichung zur Anpassung an den jeweiligen Benutzer der Vorrichtung erforderlich sein. $f_2(\Delta T, VO_2)$ gibt für den Fettstoffwechsel die funktionale Abhängigkeit der Glukosekonzentration von der Wärmeproduktion und vom Sauerstoffverbrauch an.

Die erfindungsgemäße Sensoreinheit kann (in Kombination mit der oben erwähnten integrierten oder separaten Auswertungseinheit) somit zur Bestimmung der lokalen Glukosekonzentration aus dem lokalen Sauerstoffverbrauch und der lokalen Wärmeproduktion verwendet werden. Hierzu muss die Sensoreinheit die geeigneten Messmodalitäten aufweisen. Die Ermittlung des Sauerstoffverbrauchs, kann, wie oben erläutert, durch die Kombination der Oximetrie mit der bioelektrischen Impedanzmessung erfolgen. Zur Ermittlung der Wärmeproduktion ist dann noch zusätzlich der erwähnte Wärmesensor erforderlich. Um schließlich die Glukosekonzentration nach dem oben angegebenen funktionalen Zusammenhang berechnen zu können, sollte noch der Korrekturfaktor X, beispielsweise aus dem zeitlichen Verlauf der Pulswellengeschwindigkeit, ermittelt werden. Dies kann, wie ebenfalls oben erläutert, durch kombinierte Messung von EKG-Signalen und plethysmographischen Signalen erfolgen. Zur Bestimmung der Glukosekonzentration sind also zweckmäßigerweise in der erfindungsgemäßen Sensoreinheit ein Pulsoximeter, eine EKG-Einheit, eine bioelektrische Impedanzmesseinheit sowie ein Wärmesensor kombiniert.

Die zuvor skizzierte Methode erlaubt zunächst nur eine Bestimmung der intrazellulären Glukosekonzentration. Mit der Blutglukosekonzentration besteht vereinfacht der folgende Zusammenhang:

$$[Glu]_{Zelle} = a + b * \ln (c * [Glu]_{Blut})$$

Die Konstanten a, b und c hängen von der individuellen Physiologie des untersuchten Patienten ab. Somit kann die mit der Sensoreinheit verbundene Auswertungseinheit weiterhin eingerichtet sein zur Bestimmung des Blutglukosespiegels aus der lokalen Glukosekonzentration, wobei von der Physiologie des Patienten abhängige Parameter berücksichtigt werden müssen. Diese Parameter können durch entsprechende Eichung bestimmt werden, beispielsweise durch Vergleich mit in herkömmlicher Weise invasiv bestimmten Blutglukosewerten.

Für die praktische Nutzung kann die erfindungsgemäße Sensoreinheit an ein beliebiges programmgesteuertes Gerät, beispielsweise einen Computer, ein Mobiltelefon, ein Handheld, etc. angeschlossen werden, wobei die Funktionen zur Auswertung der erfassten Messsignale durch auf dem programmgesteuerten Gerät ablaufende Software realisiert sind. Aufgrund der geringen Größe der Sensoreinheit kann diese auch in ein beliebiges Accessoire, wie z.B. eine Brille, eine Armbanduhr, ein Schmuckstück oder dergleichen, oder in ein Kleidungsstück (sog. "Smart Clothes") integriert werden. Bei dieser Ausgestaltung wird z.B. die bei dem programmgesteuerten Gerät ohnehin vorhandene Datenverarbeitungselektronik zur Verarbeitung der mittels der Sensoreinheit gewonnenen Messsignale benutzt. Dies lässt sich durch Bereitstellung entsprechender Software leicht bewerkstelligen. Gleichzeitig können die mittels der Software ermittelten diagnostischen Daten gespeichert werden. Dies ermöglicht es, den Verlauf einer Erkrankung und die Effekte einer entsprechenden Therapie zu verfolgen und zu dokumentieren. Sinnvollerweise kann auch eine Datenfernübertragung der mittels der Sensoreinheit erfassten und ausgewerteten diagnostischen Daten erfolgen. Die Datenübertragung kann z.B. über ein Datennetz (z.B. Internet) erfolgen. Alternativ können die diagnostischen Daten über ein Mobilfunknetz übermittelt werden, wenn die Sensoreinheit gemäß der Erfindung z.B. in ein Mobiltelefon integriert ist. Die rohen Messsignale oder die ausgewerteten diagnostischen Daten können zum Beispiel an eine zentrale Stelle ("Healthcare-Center") zur eingehenderen Analyse und Dokumentation sowie zur Überwachung der zeitlichen Entwicklung einzelner Werte übermittelt werden. Dort werden die Daten z.B. mittels geeigneter Analysealgorithmen ggf. unter Berücksichtigung von hinterlegten Patientendaten (einschließlich Informationen bezüglich chronischer Erkrankungen oder Vorerkrankungen) ausgewertet. Das Ergebnis kann wiederum über das jeweilige Daten- oder Kommunikationsnetz z.B. an das Mobiltelefon zurück gesendet werden, um den Benutzer der Vorrichtung entsprechend über seinen Gesundheitszustand zu informieren. Von der zentralen Stelle aus können auch bei Bedarf weitere gezielte Messungen mittels der erfindungsgemäßen Sensoreinheit initiiert werden. Außerdem können zum Zwecke einer erweiterten Anamnese veranlasst durch die Auswertungsergebnisse Rückfragen an den Patienten über das Daten- oder Kommunikationsnetz übermittelt werden. Die Daten und Auswertungsergebnisse können automatisch an einen behandelnden Arzt übermittelt werden. Falls sich aus den Mess- und Auswertungsergebnissen Hinweise auf einen medizinischen Notfall ergeben, können die erforderlichen Maßnahmen (z.B. automatische Alarmierung des Rettungsdienstes) sofort in die Wege geleitet werden. Ein weiterer Vorteil der Datenfernübertragung ist, dass die erforderliche Software zur Auswertung der Messsignale nicht in der Vorrichtung selbst implementiert sein muss, sondern lediglich an der zentralen Stelle, wo die Daten empfangen werden, vorgehalten und gepflegt werden muss.

Bei pulsoximetrischen Messungen hat der Anpressdruck des Körpergewebes (z.B. des Fingers) auf den optischen Sensor signifikanten Einfluss auf die Messsignale. Demzufolge kann es sinnvoll sein, die erfindungsgemäße Sensoreinheit mit Mitteln zur Bestimmung des Anpressdrucks des Körpergewebes auszustatten. Es kann sich dabei um einen herkömmlichen Drucksensor, beispielsweise in Form eines piezoresistiven Elements handeln. Ebenso möglich sind optische Verfahren zur Bestimmung des Anpressdrucks. Denkbar ist es auch, den Anpressdruck aus den (pulsoximetrischen) Signalen selbst zu bestimmen, da sich der Anpressdruck charakteristisch auf die Messsignale auswirkt. Der ermittelte Anpressdruck kann dann bei der weiteren Auswertung der Messsignale berücksichtigt werden, um den Einfluss des Anpressdrucks beispielsweise auf die Durchblutung zu kompensieren.

Gemäß der Erfindung sind die optische Messeinheit, die EKG-Einheit und ggf. der Temperatur- oder Wärmesensor in einem gemeinsamen Sensorgehäuse untergebracht. An der Oberseite des Sensorgehäuses ist eine flächige EKG-Elektrode, beispielsweise in Form einer elektrisch leitenden Folie oder eines elektrisch leitenden Blechs, ausgebildet, die wenigstens eine Ausnehmung für den Durchtritt der von der wenigstens einen Strahlungsquelle emittierten Strahlung aufweist. Die flächige EKG-Elektrode weist eine weitere Ausnehmung für den Temperatur- oder Wärmesensor auf. Die Strahlungsquellen, der Strahlungssensor und der Temperatur- oder Wärmesensor und die bioelektrische Impedanzmesseinheit können auf einer gemeinsamen Platine innerhalb des Sensorgehäuses angeordnet sein. Somit sind die benötigten Messmodalitäten in dem Sensorgehäuse zusammengefasst, welches eine Einheit bildet, die in ein beliebiges Diagnosegerät problemlos und flexibel integrierbar ist. Das Sensorgehäuse hat Abmessungen von weniger als 1 cm x 1 cm x 1 cm, um problemlos und flexibel im Sinne der Erfindung eingesetzt werden zu können. An der Oberseite des Sensorgehäuses kann außerdem wenigstens eine zusätzliche flächige Elektrode ausgebildet sein, die als Einspeise- oder Messelektrode der Impedanzmesseinheit dient, um zusätzlich eine bioelektrische Impedanzmessung zu ermöglichen. Dabei ist es sinnvoll, die ohnehin vorhandenen EKG-Elektroden auch als Einspeise- oder Messelektroden für die Bioimpedanzmessung zu benutzen. Ingesamt ergibt sich eine äußerst kompakte integrierte Sensoreinheit, die verschiedene Messmodalitäten enthält. Von sämtlichen Messmodalitäten kann derselbe Bereich des zu untersuchenden Körpergewebes (z.B. eine die Oberfläche des Sensorgehäuses berührende Fingerspitze eines Patienten) erfasst werden, um, wie oben erläutert, den Metabolismus und das Herz-/Kreislaufsystem des Patienten gleichzeitig zu untersuchen. Dies gestaltet die Durchführung einer Messung äußerst einfach und effektiv.

Ausführungsbeispiele der Erfindung werden im Folgenden unter Bezugnahme auf die Zeichnungen näher erläutert. Es zeigen:

Figur 1    Schematische Ansicht der Integration der erfindungsgemäßen Sensoreinheit in eine Computertastatu r;

Figur 2    Darstellung der Funktion der erfindungsgemäßen Sensoreinheit anhand eines Blockdiagramms;

Figur 3    schematische Ansicht der Integration der Sensoreinheit in ein Mobiltelefon;

Figur 4    Illustration der diagnostischen Sensoreinheit;

Figur 5    lichtleitendes Element der erfindungsgemäßen Sensoreinheit;

Figur 6    Draufsicht auf ein weiteres Ausführungsbeispiel der erfindungsgemäßen Sensoreinheit.

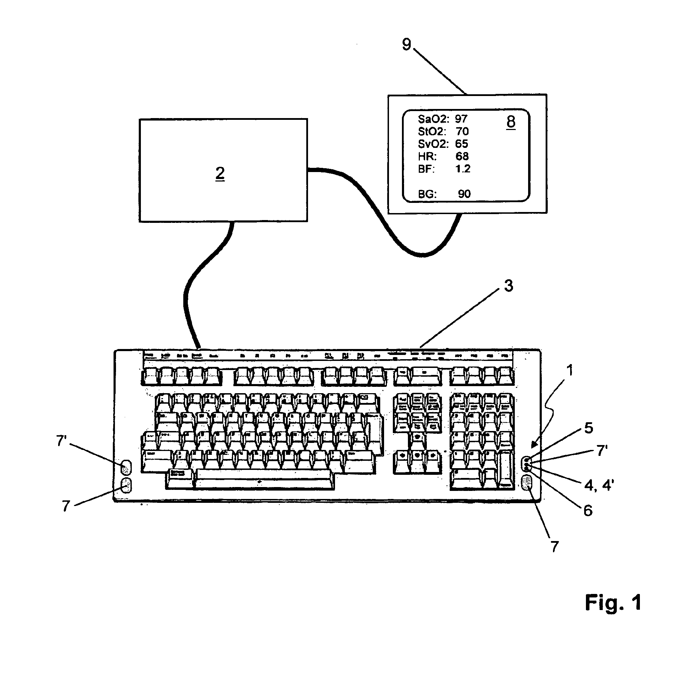

Die Figur 1 zeigt eine insgesamt mit der Bezugsziffer 1 bezeichnete Sensoreinheit gemäß der Erfindung, die in ein Computersystem bestehend aus Computer 2 und Tastatur 3 integriert ist. Die Sensoreinheit 1 weist verschiedene Messmodalitäten auf, die an der Bedienoberfläche der Tastatur 3 zugänglich sind. Diese berührt der Benutzer des Computersystems zur Durchführung einer Messung mit den Fingerspitzen. In die Sensoreinheit 1 sind Lichtquellen 4, 4' beispielsweise in Form von Leuchtdioden integriert, die dazu in der Lage ist, Licht bei verschiedenen Wellenlängen zu emittieren. Hierzu sind verschiedene lichtemittierende Halbleiterelemente in einem gemeinsamen Sensorgehäuse (in Figur 1 nicht dargestellt) untergebracht. Ebenso denkbar ist die Verwendung von Lichtwellenleitern, um das Licht von verschiedenen Lichtquellen an die Bedienoberfläche der Tastatur 3 zu führen. Des Weiteren umfasst die Sensoreinheit 1 einen oder mehrere Fotosensoren 5. Die Fotosensoren sind in unmittelbarer Nähe zur Lichtquelle 4 bzw. 4' angeordnet. Die Sensoren 5 empfangen das im Gewebe an der Fingerspitze des Benutzers gestreute Licht der Lichtquelle 4 bzw. 4'. Weiterhin ist unmittelbar neben der Lichtquelle 4 bzw. 4' ein Wärmesensor 6 vorgesehen. Dadurch ist gewährleistet, dass die Bestimmung der Durchblutung anhand der Wärmemessung am selben Messort erfolgt wie die optische Messung. Außerdem sind an der Oberfläche der Sensoreinheit 1 insgesamt vier Elektroden 7 bzw. 7' zur Messung der bioelektrischen Impedanz vorgesehen. Der Benutzer der Vorrichtung berührt mit einer Hand jeweils zwei Elektroden 7 bzw. 7' gleichzeitig. Eine der beiden Kontaktflächen dient zur Aufprägung eines elektrischen Stromes am Messort, während die andere Kontaktfläche zur Spannungsmessung genutzt wird. Auf diese Weise ist sichergestellt, dass die Messergebnisse nicht von den Kontaktwiderständen der Messelektroden beeinflusst sind. Die beiden mit der Bezugsziffer 7 bezeichneten Elektroden werden außerdem als EKG-Elektroden einer ebenfalls in die Sensoreinheit 1 integrierten EKG-Einheit verwendet. Die beiden Elektroden werden jeweils mit den Fingerspitzen berührt, so dass sich eine Zweipunkt-Ableitung (Arm-zu-Arm-Messung) ergibt. Die mittels der in die Tastatur 3 integrierten Sensoreinheit 1 aufgenommenen Messsignale werden mittels des Computers 2 verarbeitet. Die so gewonnenen physiologischen Parameter werden dann auf einer Anzeigefläche 8 eines an den Computer 2 angeschlossenen Monitors 9 ausgegeben. Angezeigt werden z. B. die arterielle ($SaO_2$), die kapillare ($StO_2$) und die venöse ($SvO_2$) Sauerstoffsättigung. Angezeigt wird weiterhin die ermittelte Herzfrequenz (HR), der Fettgehalt des Gewebes (BF). Schließlich wird noch ein Blutglukosewert (BG) angezeigt. Der Benutzer kann jederzeit die ihn interessierenden physiologischen Parameter ermitteln. Hierzu legt er lediglich die Finger, mit denen er ansonsten die Tasten der Tastatur 3 betätigt, auf die Elektroden 7, 7'. Die Parameter werden dann nach der Verarbeitung der Messsignale mittels des Computers 2 sofort mittels des Monitors 9 angezeigt. Der Benutzer der Vorrichtung 1 muss seine Arbeit am Computer 2 also zur Ermittlung der physiologischen Parameter praktisch nicht unterbrechen.

Bei dem in der Figur 1 dargestellten Ausführungsbeispiel der Sensoreinheit 1 sind zwei Strahlungsquellen 4 und 4' vorgesehen, welche unterschiedliche Volumenbereiche des untersuchten Körpergewebes bestrahlen. Hierzu haben die zwei Strahlungsquellen 4 und 4' unterschiedliche räumliche Abstrahlcharakteristiken, nämlich unterschiedliche Abstrahlwinkel. Bei der Strahlungsquelle 4 handelt es sich um eine Leuchtdiode, während es sich bei der Strahlungsquelle 4' um einen Laser, beispielsweise einen so genannten VCSEL-Laser (engl. "vertical cavity surface emitting laser") handelt. Sowohl die Leuchtdiode 4 als auch der Laser 4' emittieren Licht mit sehr ähnlicher Wellenlänge (z. B. 630 nm und 650 nm), aber mit unterschiedlichen Öffnungswinkeln (z. B. 25° und 55°). Mit der in der Figur 1 dargestellten Anordnung ist - wie oben beschrieben - eine differenzielle Messung von Metabolismus-induzierten Änderungen des Sauerstoffgehalts im Blut möglich. Hierzu muss die Wellenlänge der von den beiden Strahlungsquellen 4 und 4' jeweils emittierten Strahlung in einem Bereich liegen, in welchem das Licht von Oxihämoglobin und Desoxihämoglobin unterschiedlich stark absorbiert wird. Für eine Absolutmessung des Sauerstoffgehalts des Blutes (Sauerstoffsättigung) müssen weitere Strahlungsquellen (in der Figur 1 nicht dargestellt) vorhanden sein, deren Lichtwellenlänge in einem Spektralbereich liegt, in welchem die Lichtabsorption von Oxihämoglobin und Desoxihämoglobin im Wesentlichen gleich ist (so genannter isobektischer Punkt). Das von der Leuchtdiode bzw. von dem Laser emittierte Licht kann mittels entsprechender Lichtleitfasern an die entsprechende Stelle an der Bedienoberfläche der Tastatur geführt werden. In diesem Fall sind mit den Bezugsziffern 4 und 4' in der Figur 1 die entsprechenden Faserenden dargestellt. Es ist möglich, die Leuchtdiode und den Laser so an die entsprechenden Fasern anzukoppeln, dass sie mit dem gewünschten unterschiedlichen Öffnungswinkel in das zu untersuchende Körpergewebe einstrahlen. Dementsprechend werden mit beiden Strahlungsquellen unterschiedliche Volumina des Körpergewebes untersucht. Aufgrund des größeren Öffnungswinkels ist der Anteil der nicht-durchbluteten Epidermis an dem mittels der Leuchtdiode untersuchten Körpergewebe größer als beim Laser. Das im Körpergewebe gestreute und teilweise absorbierte Licht sowohl der Strahlungsquelle 4 als auch der Strahlungsquelle 4' wird mittels der Sensoren 5 detektiert. Die Sensoren 5 müssen nicht direkt an der Oberfläche der Sensoreinheit 1 angeordnet sein. Stattdessen kann das Licht über Lichtleitfasern den im Inneren der Sensoreinheit 1 angeordneten Sensoren zugeführt werden. Zur Unterscheidung des Lichtes der Strahlungsquelle 4 von dem Licht der Strahlungsquelle 4' können die beiden Lichtquellen 4 und 4' unterschiedlich zeitlich moduliert betrieben werden, wobei die mittels der Sensoren 5 detektierten Signale entsprechend demoduliert werden. Alternativ ist es möglich, die Strahlung der beiden Strahlungsquellen 4 und 4' anhand der unterschiedlichen Wellenlänge zu unterscheiden. Die Strahlungsintensität der von den Strahlungsquellen 4 und 4' emittierten Strahlung wird mit der Weglänge beim Durchgang durch das Körpergewebe geschwächt, wobei der Zusammenhang der Intensitätsschwächung mit der Konzentration der absorbierenden Substanz (oxigeniertes Hämoglobin) durch das bekannte Lambert-Beersche Gesetz gegeben ist. Mittels der in der Figur 1 dargestellten Sensoren 5 können die interessierenden Parameter der Intensitätsschwächung bestimmt werden, und zwar getrennt für die von den Strahlungsquellen 4 und 4' jeweils erfassten Volumenbereiche des untersuchten Körpergewebes. Die den verschiedenen Strahlungsquellen 4 und 4' zuzuordnenden Parameter der Intensitätsschwächung können mittels einer geeignet programmgesteuerten Auswertungseinheit zueinander in Beziehung gesetzt werden, um auf diese Weise eine differenzielle Messung durchzuführen. Im einfachsten Fall werden aus den Parametern der Intensitätsschwächung der Strahlung der beiden Strahlungsquellen 4 und 4' jeweils Quotienten berechnet. Aus Änderungen dieser Quotienten kann dann auf Änderungen im Metabolismus zurückgeschlossen werden. Steigt beispielsweise nach der Nahrungsaufnahme der Blutglukosespiegel, gelangt (nach einer gewissen zeitlichen Verzögerung) entsprechend mehr Glukose in die Zellen des Körpergewebes und wird dort umgesetzt. Dabei wird Sauerstoff verbraucht. Diesen Sauerstoff erhalten die Zellen über das Blut. Dabei wird aus dem oxigenierten Hämoglobin durch Abgabe von Sauerstoff desoxigeniertes Hämoglobin. Dementsprechend steigt das Verhältnis von desoxigeniertem Hämoglobin zu oxigeniertem an. Aufgrund der unterschiedlichen Öffnungswinkel der Strahlung der Strahlungsquellen 4 und 4' wirken sich die Änderungen der Hämoglobinkonzentration unterschiedlich auf die jeweilige Intensitätsschwächung aus. Somit können aus den Quotienten der Parameter der Intensitätsschwächung Veränderungen der Hämoglobinkonzentration detektiert werden. Dies ermöglicht es, indirekt auf den Sauerstoffverbrauch zurückzuschließen. Da der Sauerstoffverbrauch seinerseits vom Blutglukosespiegel abhängt, kann mittels der erläuterten differenziellen Messung der Strahlungsabsorption auch der Blutglukosespiegel ermittelt werden. Als sinnvolle Ergänzung wird parallel zur optischen Messung eine Bioimpedanzanalyse durchgeführt, wozu die in der Figur 1 dargestellten Elektroden 7 und 7' vorgesehen sind. Zweck der Bioimpedanzmessung ist vor allem die Bestimmung der lokalen Durchblutung. Diese kann als weiterer Parameter bei der Bestimmung des Sauerstoffverbrauchs und damit auch des Blutglukosespiegels herangezogen werden. Unterschiedliche Öffnungswinkel der Strahlung können auch mit nur einer Strahlungsquellen 4 durch Verwendung entsprechender optischer Elemente (z.B. Strahlteiler, Linsen etc.) erzeugt werden.

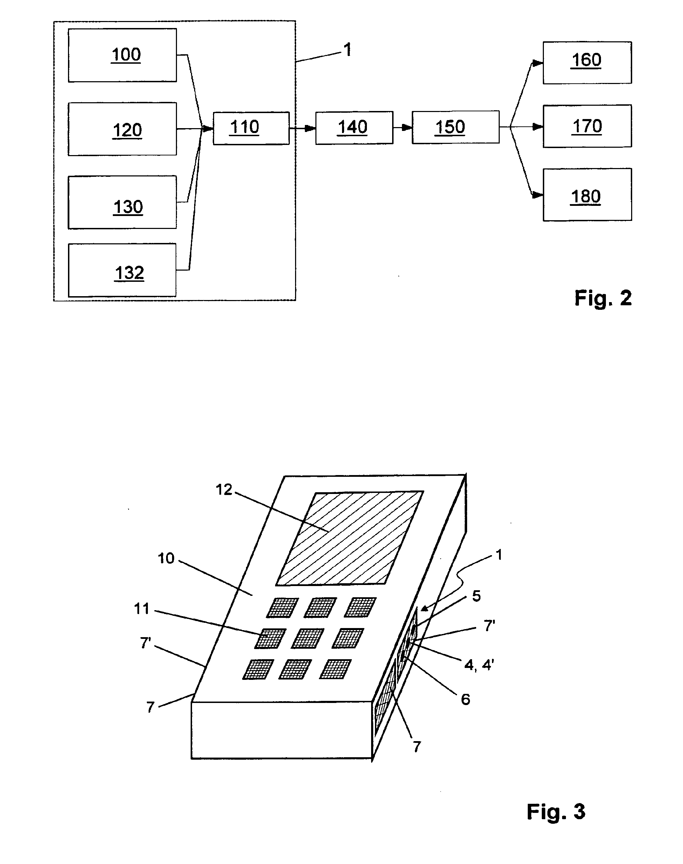

Die Figur 2 zeigt schematisch den Aufbau der erfindungsgemäßen Sensoreinheit 1 als Blockdiagramm. Die Sensoreinheit 1 umfasst eine optische Messeinheit 100 zur optischen Messung der Sauerstoffkonzentration im Blutgefäßsystem des Körpergewebes am jeweiligen Messort. Die mittels der optischen Messeinheit 100 erfassten oximetrischen und plethysmographische Signale werden einer Analyseeinheit 110 zugeführt. Eine weitere wesentliche Komponente der Vorrichtung 1 ist eine Wärmemesseinheit 120 zur Bestimmung der lokalen Wärmeproduktion. Bei der Wärmemesseinheit 120 handelt es sich um einen speziellen Wärmesensor, welcher die jeweils untersuchte Körperstelle isoliert. Diese Stelle kann somit nur noch Wärme durch den Blutstrom aufnehmen oder abgeben. Daher ist es möglich, durch die zeitaufgelöste Messung der Temperatur die Durchblutung und die Wärmeproduktion zu bestimmen. Bei einer starken Durchblutung erreicht die untersuchte Körperstelle in sehr kurzer Zeit ihre maximale Temperatur. Bei geringer Durchblutung dauert dies länger. Zusätzlich kann über die Extrapolation der gemessenen Temperatur auf die arterielle Temperatur geschlossen werden, da die Temperatur am Ort der Messung nur durch die arterielle Temperatur und durch die lokale Wärmeproduktion bestimmt wird. Auch die mittels der Wärmemesseinheit 120 erfassten Messsignale werden der Analyseeinheit 110 zur Weiterverarbeitung zugeführt. Außerdem umfasst die Sensoreinheit eine Impedanzmesseinheit 130, die zur Erfassung von lokalen Gewebeparametem mittels bioelektrischer Impedanzmessung dient. Die Messsignale der Impedanzmesseinheit 130 werden ebenfalls mittels der Analyseeinheit 110 verarbeitet. Schließlich ist gemäß der Erfindung noch eine EKG-Einheit 132 zur Erfassung eines EKG-Signals vorgesehen. Auch die EKG-Einheit 132 ist zur Verarbeitung der EKG-Signale mit der Analyseeinheit 110 verbunden. Der optischen Messeinheit 100 sind die Lichtquelle 4 sowie die Lichtsensoren 5 der in der Figur 1 dargestellten Sensoreinheit 1 zugeordnet. Die Wärmemesseinheit 120 ist mit dem Wärmesensor 6 verbunden. Die Impedanzmesseinheit 130 erfasst Messsignale über die Elektroden 7 bzw. 7' der Sensoreinheit 1. Die Analyseeinheit 110 führt eine Vorverarbeitung sämtlicher Messsignale durch. Hierzu durchlaufen die Signale ein Bandpass-Filter, um Störungen im Bereich der Netzfrequenz von 50 bzw. 60 Hz herauszufiltern. Des Weiteren werden die Signale einer Rauschunterdrückung unterzogen. Nach Passieren der Analyseeinheit 110 gelangen die aufbereiteten Signale der optischen Messeinheit 100, der Wärmemesseinheit 120, der Impedanz-Messeinheit 130 und der EKG-Einheit 132 in eine Auswertungseinheit 140. Die Auswertungseinheit 140 ist dafür zuständig, aus den Messsignalen die für die Diagnose wesentlichen Parameter zu berechnen. Die Funktionen der Auswertungseinheit 140 sind im Wesentlichen durch Software realisiert. Somit ist die Auswertungseinheit 140 bei dem gezeigten Ausführungsbeispiel nicht Bestandteil der eigentlichen Sensoreinheit 1. Aus den zeitabhängig aufgenommenen Messsignalen der Impedanzmesseinheit 130 wird zunächst die Zusammensetzung des untersuchten Körpergewebes (Wassergehalt, Fettgehalt usw.) berechnet. Aus den Signalen der optischen Messeinheit 100 wird die arterielle Sauerstoffsättigung und - unter Zugrundelegung der zuvor auf der Basis der Impedanzmessung ermittelten Gewebeparameter - die kapillare Sauerstoffsättigung berechnet. Weiterhin werden aus den Messsignalen der Wärmemesseinheit 120 und aus den plethysmographischen Daten, die aus der zeitabhängigen Impedanzmessung ableitbar sind, die Durchblutung und die arterielle Temperatur bestimmt. Aus den Signalen der EKG-Einheit 132 und denjenigen der optischen Messeinheit 100 wird die Pulswellengeschwindigkeit bestimmt. Schließlich werden mittels der Auswertungseinheit 140 aus den Ergebnissen sämtlicher zuvor durchgeführter Berechnungen die venöse Sauerstoffsättigung, und daraus weitere metabolische Parameter, insbesondere der lokale Sauerstoffverbrauch und die Glukosekonzentration am Messort berechnet. Die Berechnungsergebnisse werden mittels einer Diagnoseeinheit 150 interpretiert. Die Diagnoseeinheit 150, die ebenfalls als Software auf dem Computer 2 implementiert ist, dient zur Bewertung der mittels der Auswertungseinheit 140 berechneten lokalen metabolischen Parameter. Die Auswertungseinheit 140 und die Diagnoseeinheit 150 sind zur Anzeige der Messresultate mit einer Grafikeinheit 160 verbunden, die ihrerseits den Monitor 9 ansteuert. Die gewonnenen Daten sind in einer Speichereinheit 170 speicherbar, und zwar unter gleichzeitiger Speicherung des Datums und der Uhrzeit der jeweiligen Messung. Außerdem ist eine Schnittstelleneinheit 180 vorgesehen, die zur Verbindung des Computers 2 mit einem Datennetzwerk zur Übertragung der berechneten physiologischen Parameter dient. Über die Schnittstelleneinheit 180 können sämtliche Daten und Parameter, insbesondere auch die in der Speichereinheit 170 gespeicherten Daten und Parameter, an einen nicht näher dargestellten PC eines behandelnden Arztes übertragen werden. Dort können die Daten detaillierter analysiert werden. Insbesondere können über einen längeren Zeitraum mit der Sensoreinheit 1 aufgenommene Daten und Parameter auf Veränderungen hin untersucht werden, um daraus Schlussfolgerungen hinsichtlich der Entwicklung einer bestehenden Erkrankung ableiten zu können.

Die Figur 3 zeigt ein zweites Einsatzbeispiel für die erfindungsgemäße Sensoreinheit 1, nämlich in einem Mobiltelefon 10. An der Vorderseite des Gerätes 10 sind die üblichen Bedientasten 11 zu erkennen. In die seitlichen Flächen des Gehäuses des Gerätes 10 sind bündig die diagnostischen Messsensoren der Sensoreinheit 1 integriert. Diese berührt der Benutzer des Mobiltelefons 10 zur Durchführung einer Messung mit den Fingern. An den seitlichen Gehäuseoberflächen des Mobiltelefons 10 sind insgesamt vier Elektroden 7 bzw. 7' zur Messung der bioelektrischen Impedanz vorgesehen. Der Benutzer des Mobiltelefons 10 berührt mit einer Hand jeweils zwei Elektroden 7 bzw. 7' gleichzeitig. Die beiden Elektroden werden jeweils mit den Fingerspitzen berührt, so dass sich eine Zweipunkt-Ableitung (Arm-zu-Arm-Messung) ergibt. Die mittels der verschiedenen in die Sensoreinheit 1 des Mobiltelefons 10 integrierten Sensoren aufgenommenen Messsignale werden mittels des (nicht näher dargestellten) Mikroprozessors des Mobiltelefons 10 verarbeitet. Die so gewonnenen physiologischen Parameter werden dann auf einem Display 12 des Mobiltelefons 10 ausgegeben. Der Benutzer kann jederzeit die ihn interessierenden physiologischen Parameter ermitteln. Hierzu legt er lediglich die Finger, mit denen er ansonsten die Tasten 11 betätigt, auf die Elektroden 7, 7'. Die Softwaresteuerung des Mobiltelefons 10 erkennt die Berührung automatisch und startet die Messung. Die Parameter werden dann nach der Verarbeitung der Messsignale mittels des Mikroprozessors des Mobiltelefons 10 sofort mittels des Displays 12 angezeigt. Die Funktion des durch Integration der Sensoreinheit 1 als medizinische Messvorrichtung ausgebildeten Mobiltelefons 10 basiert wesentlich auf dem oben beschriebenen indirekten Verfahren zur nicht-invasiven Bestimmung des Blutglukosewertes, bei welchem die Wirkung der Glukose bzw. der Energieumsatz der durch Glukose initiierten physiologischen Reaktionen im Körper untersucht wird. Auf die entsprechende Beschreibung zur Erläuterung des in der Figur 1 gezeigten Ausführungsbeispiels wird verwiesen. Ähnlich wie bei der Tastatur 3 müssen die Lichtquellen 4, 4' und die Sensoren 5 auch bei dem Mobiltelefon 10 nicht direkt an der Gehäuseoberfläche angeordnet sein. Stattdessen kann das Licht über Lichtleitfasern von bzw. zur Gehäuseoberfläche geführt werden, wobei sich die eigentlichen Lichtquellen bzw. Sensoren im Inneren des Gehäuses befinden. Mehrere Lichtquellen bzw. Sensoren können an eine einzige Lichtleitfaser gekoppelt sein.

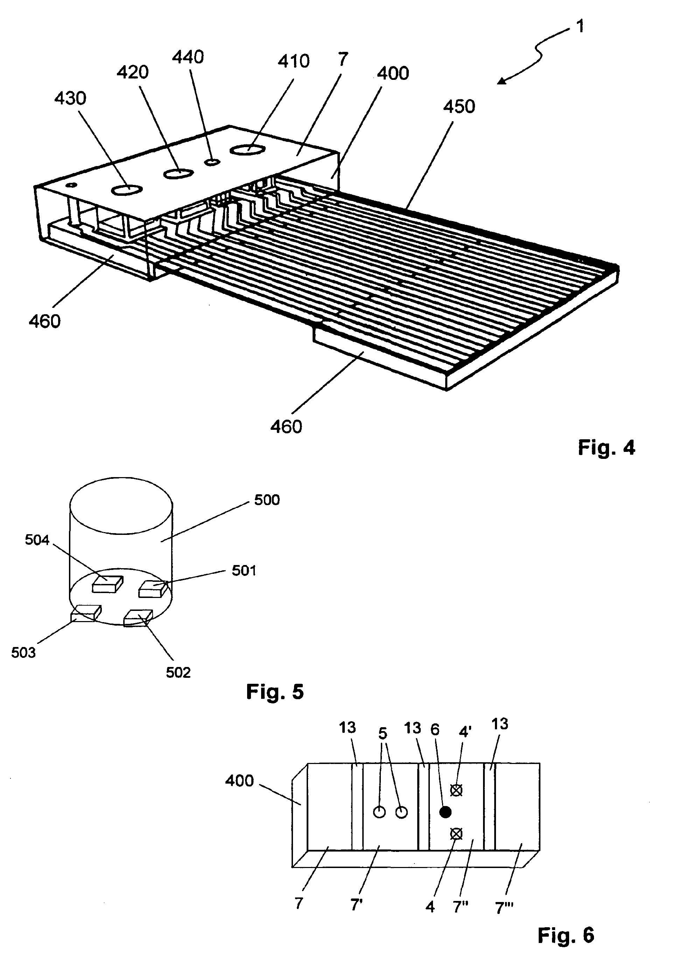

Die Figur 4 illustriert die Konstruktion der diagnostischen Sensoreinheit 1 gemäß der Erfindung. Die verschiedenen Messeinheiten der Sensoreinheit 1 sind in ein Sensorgehäuse 400 mit sehr kleinen äußeren Abmessungen integriert. An der Oberseite des Gehäuses 400 ist eine flächige EKG-Elektrode 7 angeordnet, die aus einer dünnen, elektrisch leitenden Folie besteht. Beim Einbau der Sensoreinheit in eine Computertastatur oder in ein mobiles Gerät wird das Sensorgehäuse 400 so angeordnet, dass der Benutzer die EKG-Elektrode 7 und eine weitere Elektrode (in der Figur 4 nicht dargestellt) zur EKG-Ableitung mit verschiedenen Extremitäten berühren kann. Sinnvollerweise ist die EKG-Elektrode eine dünne Edelstahlfolie. Die kleine Bauform des Mikro-Gehäuses von 5 mm (B) × 8 mm (L) × 1,8 mm (H) bei dem dargestellten Ausführungsbeispiel ermöglicht einen flexiblen und kostengünstigen Einbau der Sensoreinheit in unterschiedliche Gehäuse verschiedener am Markt vorhandener Geräte. Zur gleichzeitigen Bestimmung der Sauerstoffsättigung in arteriellem Blut ist in das Sensorgehäuse 400 eine optische Messeinheit, nämlich ein Pulsoximeter integriert. Dieses umfasst zwei oder mehr optische Strahlungsquellen, deren Strahlung durch eine Ausnehmung 410 in der EKG-Elektrode 7 hindurch treten kann. Außerdem umfasst das Pulsoximeter zwei optische Strahlungssensoren, z.B. in Form von Photodioden. Das im Körpergewebe (z.B. eines auf die Elektrode 7 aufgelegten Fingers) gestreute Licht fällt durch zwei Ausnehmungen 420 und 430 in der Elektrode 7 auf die Strahlungssensoren. Die Ausnehmungen 420 und 430 sind in unterschiedlichem Abstand zur Ausnehmung 410 angeordnet. In der Sensoreinheit wird das Licht von zwei oder mehr optischen Strahlungsquellen (z.B. Leuchtdioden) innerhalb des Gehäuses 400 in eine Lichtleitfaser oder in einen geeigneten lichtleitenden Körper gekoppelt, so dass sich an der Oberseite des Mikro-Gehäuses nur eine Ausnehmung 410 für sämtliche Strahlungsquellen befindet und das Licht sämtlicher Strahlungsquellen der Sensoreinheit an derselben Stelle in das zu untersuchende Körpergewebe geleitet wird. Die Photodioden sind jeweils einzeln an eine Lichtleitfaser oder an einen geeignet ausgebildeten lichtleitenden Körper gekoppelt Die optische Messeinheit ermöglicht die gleichzeitige Messung der Sauerstoffsättigung des in dem untersuchten Körpergewebe zirkulierenden Blutes und des Volumenpulses. Sinnvollerweise werden hierfür nicht nur Leuchtdioden, sondern auch andere Strahlungsquellen, wie z.B. Vertical Cavity Surface Emitting Laser (VCSEL), benutzt. Zur gleichzeitigen Bestimmung der thermischen Eigenschaften des untersuchten Gewebes ist in das Sensorgehäuse ein Temperatursensor, nämlich ein Thermistor integriert. Für diesen ist eine weitere Ausnehmung 440 in der EKG-Elektrode 7 vorgesehen. Der Thermistor ist in dem Sensorgehäuse 400 derart angeordnet, dass er guten thermischen Kontakt zum untersuchten Körpergewebe hat. Bei dem dargestellten Ausführungsbeispiel befindet sich der Thermistor zwischen der Ausnehmung 410 für die Lichtleitfaser der optischen Strahlungsquellen und der Ausnehmung 420 für die Lichtleitfaser der ersten Photodiode. Die Sensoreinheit kann problemlos durch eine Impedanzmesseinheit ergänzt werden. Hierzu muss an der Oberseite des Sensorgehäuses 400 wenigstens eine zusätzliche flächige Elektrode (in der Figur 4 nicht dargestellt) ausgebildet sein, die dann als Einspeise- oder Messelektrode der Impedanzmesseinheit dient. Sinnvollerweise können dieselben Messelektroden zur Erfassung des Bioimpedanzsignals und des EKG-Signals benutzt werden. Für den elektrischen Kontakt der Sensoreinheit (z.B. mit der Elektronik eines Mobiltelefons) ist das Sensorgehäuse 400 mit allen integrierten Messeinheiten direkt auf ein Flachbandkabel 450 mit geeigneter Leiterbahnführung montiert, so dass eine einfache elektrische Montage der Sensoreinheit 1 mit Hilfe des Flachbandkabels 450 möglich ist. Das Flachbandkabel 450 kann zu Stabilisierungszwecken an geeigneten Stellen Versteifungen 460 aufweisen.

Die Figur 5 zeigt das oben in Bezug auf die Figur 4 erwähnte lichtleitende Element 500 mit insgesamt vier an der Unterseite des Elementes 500 angekoppelten LED-Chips 501, 502, 503 und 504, die Lichtquellen der optischen Messeinheit der erfindungsgemäßen Sensoreinheit 1 bilden. Durch das eine einzige lichtleitende Element 500 wird die emittierte Strahlung sämtlicher LEDs 501, 502, 503 und 504 zur Oberfläche des Sensorgehäuses 400 geführt. Die vier LEDs 501, 502, 503 und 504 sind nebeneinander auf ein (nicht dargestelltes) Substrat, z.B. ein PCB, gebondet.

Die Figur 6 zeigt ein weiteres Ausführungsbeispiel der Erfindung, wobei an der Oberseite des Sensorgehäuses 400 insgesamt vier Elektroden 7, 7', 7" und 7'" angeordnet sind, die als Einspeise- und Messelektroden zur (lokalen) bioelektrischen Impedanzmessung sowie zur EKG-Ableitung nutzbar sind. Die Elektroden 7, 7', 7" und 7'" sind durch elektrisch isolierende Streifen 13 voneinander getrennt.

## Patentansprüche

1. Diagnostische Sensoreinheit (1) zur nicht-invasiven Erfassung von wenigstens einem physiologischen Parameter von hautoberflächennahem Körpergewebe,

   - mit einer optische Messeinheit (100), die zwei oder mehr Strahlungsquellen (4) zur Bestrahlung des zu untersuchenden Körpergewebes und wenigstens einen Strahlungssensor (5) zur Detektion der von dem Körpergewebe gestreuten und/oder transmittierten Strahlung umfasst,
   - mit einer EKG-Einheit (132) zur Erfassung eines EKG-Signals über zwei oder mehr EKG-Elektroden (7),
   - mit einem Temperatur- oder Wärmemesseinheit (120), die mit einem Temperatur- oder Wärmesensor (6) verbunden ist,
   - mit einer bioelektrische Impedanzmesseinheit (130),
   - mit einem Sensorgehäuse (400),
   wobei
   - die zwei oder mehr Strahlungsquellen (4), der wenigstens eine Strahlungssensor (5), die EKG-Einheit (132), die Temperatur- oder Wärmemesseinheit (120) und die bioelektrische Impedanzmesseinheit (130) in dem Sensorgehäuse (400) angeordnet sind,
   - an der Gehäuseoberfläche des Sensorgehäuses (400) wenigstens eine EKG-Elektrode (7) der EKG-Einheit (132) angeordnet ist, in der Weise, dass die EKG-Elektrode (7) die Hautoberfläche in dem von der optischen Messeinheit (100) erfassten Bereich des Körpergewebes berührt, und
   - an der Gehäuseoberfläche des Sensorgehäuses (400) wenigstens eine Einspeise- oder Messelektrode der Impedanzmesseinheit (130) angeordnet ist, **dadurch gekennzeichnet, dass**
   - die wenigstens eine EKG-Elektrode (7) flächig und als Blech aus elektrisch leitendem Material ausgebildet ist,
   - die EKG-Elektrode (7) wenigstens eine Ausnehmung (410) für den Durchtritt der von den zwei oder mehr Strahlungsquellen (4) emittierten Strahlung in das zu untersuchende Körpergewebe, eine Ausnehmung (420, 430) für den Durchtritt des im Körpergewebe gestreuten Lichtes und eine weitere Ausnehmung (440) für den Temperatur- oder Wärmesensor (6) aufweist,
   - das die zwei oder mehr Strahlungsquellen (4) mit einem lichtleitenden Element (500) verbunden sind, welches die Strahlung der wenigstens zwei Strahlungsquellen (4, 4') zur Oberfläche des Sensorgehäuses (400) leitet, und
   - die zwei oder mehr Strahlungsquellen (4), der wenigstens eine Strahlungssensor (5) und der Temperatur- oder Wärmesensor (6) auf einer gemeinsamen Platine innerhalb des Sensorgehäuses (400) angeordnet sind.

2. Diagnostische Sensoreinheit (1) nach Anspruch 1, **dadurch gekennzeichnet, dass** die Sensoreinheit (1) eine Auswertungseinheit als integralen Bestandteil aufweist, die mit den Messeinheiten (100, 132, 120, 130) verbunden ist und mit der sämtliche Messsignale der Messeinheiten (100, 132, 120, 130) auswertbar sind.

3. Diagnostische Sensoreinheit (1) nach Anspruch 1, **dadurch gekennzeichnet, dass** eine separate Auswertungseinheit (140) vorgesehen ist, wobei sämtliche Messsignale der Messeinheiten (100, 132, 120, 130) über eine geeignete Datenverbindung von der Sensoreinheit (1) an die Auswertungseinheit (140) übertragbar sind.

4. Diagnostische Sensoreinheit (1) nach Anspruch 3, **gekennzeichnet durch** eine elektrische Steckverbindung, über welche die Sensoreinheit (1) mit einem Gerät (10) der Unterhaltungs- oder Kommunikationstechnik oder mit einem anderweitigen tragbaren Gerät oder Accessoire verbindbar ist

5. Diagnostische Sensoreinheit (1) nach Anspruch 3 oder 4, **dadurch gekennzeichnet, dass** das Sensorgehäuse (400) mit allen integrierten Messeinheiten (100, 132, 120, 130) direkt auf einem Flachbandkabel (450) mit geeigneter Leiterbahnführung montiert ist.

6. Diagnostische Sensoreinheit (1) nach Anspruch 5, **dadurch gekennzeichnet, dass** das Flachbandkabel (450) Versteifungen (460) aufweist.

7. Diagnostische Sensoreinheit (1) nach einem der vorhergehenden Ansprüche, **dadurch gekennzeich-**

**net, dass** das Sensorgehäuse (400) Abmessungen von weniger als 1 cm x 1 cm x 1cm hat.

8. Diagnostische Sensoreinheit (1) nach einem der vorhergehenden Ansprüche, **dadurch gekennzeichnet, dass** wenigstens eine der EKG-Elektroden (7) gleichzeitig Einspeise- oder Messelektrode der bioelektrischen Impedanzmesseinheit (130) ist.

9. Diagnostische Sensoreinheit (1) nach einem der vorhergehenden Ansprüche, **dadurch gekennzeichnet, dass** die optische Messeinheit (100) wenigstens zwei Strahlungssensoren (5) zur Detektion der von dem Körpergewebe gestreuten und/oder transmittierten Strahlung aufweist, wobei die Strahlungssensoren (5) in unterschiedlichem Abstand zur Strahlungsquelle (4) angeordnet sind.

10. Diagnostische Sensoreinheit (1) nach einem der vorhergehenden Ansprüche, **dadurch gekennzeichnet, dass** die zwei oder mehr Strahlungsquellen (4, 4') unterschiedliche Volumenbereiche des untersuchten Körpergewebes bestrahlen.

11. Diagnostische Sensoreinheit (1) nach Anspruch 10, **dadurch gekennzeichnet, dass** die wenigstens zwei Strahlungsquellen (4, 4') unterschiedliche räumliche Abstrahlcharakteristiken haben.

12. Diagnostische Sensoreinheit (1) nach einem der vorhergehenden Ansprüche, **gekennzeichnet durch** ein Mittel zur Bestimmung des Anpressdrucks des Körpergewebes auf die Oberfläche des Sensorgehäuses (400).

13. Diagnostische Sensoreinheit (1) nach einem der Ansprüche 4 bis 12, **dadurch gekennzeichnet, dass** das Gerät (10) ein mobiles Gerät, insbesondere ein Notebook, ein Laptop, ein Palmtop oder ein Handheld ist.

**Claims**

1. Diagnostic sensor unit (1) for non-invasive detection of at least one physiological parameter of body tissue close to the skin surface,

   - having an optical measurement unit (100) that comprises two or more radiation sources (4) for irradiating the body tissue to be examined, and at least one radiation sensor (5) for detecting the radiation scattered and/or transmitted by the body tissue,
   - having an ECG-unit (132) for detecting an ECG-signal by way of two or more ECG-electrodes (7),
   - having a temperature- or heat measurement unit (120) connected with a temperature-or heat sensor (6),
   - having a bioelectrical impedance measurement unit (130),
   - having a sensor housing (400), whereby
   - the two or more radiation sources (4), the at least one radiation sensor (5), the ECG unit (132), the temperature or heat measurement unit (120) and the bioelectrical impedance measurement unit (130) are disposed in a common sensor housing (400),
   - whereby at least one ECG-electrode (7) of the ECG unit (132) is disposed on the housing surface of the sensor housing (400), in such a way, that the ECG-electrode (7) touches the skin surface in the region of the body tissue covered by the optical measurement unit (100), and
   - whereby at least one feed or measurement electrode of the impedance measurement unit (130) is disposed on the housing surface of the sensor housing (400),
   **characterized by** that
   the at least one ECG-electrode (7) is configured as a planar foil or sheet of electrically conductive material,
   - the ECG-electrode (7) has at least one recess (410) for passage of the radiation emitted by the two or more radiation sources (4) into the body tissue to be examined, a recess (420, 430) for passage of the scattered light in the body tissue and one other recess (440) for the temperature and heat sensor (6),
   - the two or more radiation sources (4) are connected with a light-conducting element (500) that guides the radiation of the at least two radiation sources (4, 4') to the surface of the sensor housing (400), and
   - the two or more radiation sources (4), the at least one radiation sensor (5) and the temperature or heat sensor (6) are disposed on a common board within the sensor housing (400).

2. Diagnostic sensor unit (1) according to claim 1, **characterized by** that

   - the sensor unit (1) has an evaluation unit (40) as an integral part of it, connected with the measurement units (100, 132, 120, 130), and by means of the evaluation unit (140) all measurement signals of the measurement units (100, 132, 120, 130) can be evaluated.

3. Diagnostic sensor unit (1) according to claim 1, **characterized by** that

   - there is a separate evaluation unit (140), whereby all measurement signals of the measurement units (100, 132, 120, 130) are transmittable from the sensor unit (1) to the evaluation unit (140) by a suitable data connection.

4. Diagnostic sensor unit (1) according to claim 3, **characterized by** an electrical plug-in connection by way of which the sensor unit (1) can be connected with a device (10) of entertainment or communications technology, or with some other portable device or accessory.

5. Diagnostic sensor unit (1) according to claim 3 or 4, **characterized by** that the sensor housing (400) together with all integrated measurement units (100, 132, 120, 130) is mounted directly on to a ribbon cable (450) with a suitable conductor track.

6. Diagnostic sensor unit (1) according to claim 5, **characterized by** that the ribbon cable (450) has reinforcements (460).

7. Diagnostic sensor unit (1) according to one of the preceding claims, **characterized by** that the sensor housing (400) has dimensions of less than 1 cm x 1 cm x 1 cm.

8. Diagnostic sensor unit (1) according to one of the preceding claims, **characterized by** that at least one of the ECG-electrodes (7) is feed or measurement electrode of the bioelectrical measurement unit (130) at the same time.

9. Diagnostic sensor unit (1) according to one of the preceding claims, **characterized by** that the optical measurement unit (100) has at least two radiation sensors (5) for detection of the radiation scattered and/or transmitted by the body tissue, whereby the radiation sensors (5) are disposed at different distances from the radiation source (4).

10. Diagnostic sensor unit (1) according to one of the preceding claims, **characterized by** that the two or more radiation sources (4, 4') irradiate different volume regions of the body tissue being examined.

11. Diagnostic sensor unit (1) according to claim 10, **characterized by** that the at least two radiation sources (4, 4') have different spatial emission characteristics.

12. Diagnostic sensor unit (1) according to one of the preceding claims, **characterized by** means for determining the contact pressure of the body tissue on the surface of the sensor housing (400).

13. Diagnostic sensor unit (1) according to one of the claims 4 to 12, **characterized by** that the device (10) is a mobile device, particularly a note book, a laptop, a palmtop, or a handheld.

## Revendications

1. Unité de capteur de diagnostic (1) pour la mesure non invasive d'au moins un paramètre physiologique d'un tissu corporel proche de la surface de la peau

   - avec une unité de mesure optique (100) qui comprend deux sources de rayonnement (4) ou plus pour l'irradiation du tissu corporel à examiner et au moins un capteur de rayonnement (5) pour la détection du rayonnement diffusé et/ou transmis par le tissu corporel,
   - avec une unité EKG (132) pour la détection d'un signal EKG par l'intermédiaire de deux électrodes EKG (7) ou plus,
   - avec une unité de mesure de température ou de chaleur (120), reliée avec un capteur de température ou de chaleur (6),
   - avec une unité de mesure d'impédance bioélectrique (130),
   - avec un boîtier de capteur (400),
   - les deux sources de rayonnements (4) ou plus, l'au moins un capteur de rayonnement (5), l'unité EKG (132), l'unité de mesure de température ou de chaleur (120) et l'unité de mesure d'impédance bioélectrique (130) étant disposées dans le boîtier de capteur (400),
   - à la surface du boîtier de capteur (400), au moins une électrode EKG (7) de l'unité KG (132) étant disposée de façon à ce que l'électrode EKG (7) soit en contact avec la surface de la peau dans la zone du tissu corporel mesuré par l'unité de mesure optique (100), et
   - à la surface du boîtier de capteur (400), au moins une électrode d'alimentation ou de mesure de l'unité de mesure d'impédance (130) étant disposée, **caractérisée en ce que**
   - l'au moins une électrode EKG (7) est plate et conçue comme une tôle constituée d'un matériau conducteur électrique,
   - l'électrode EKG (7) présente au moins un évidement (410) pour le passage du rayonnement émis par les deux sources de rayonnement (4) ou plus dans le tissu corporel à examiner, un évidement (420, 430) pour le passage de la lumière diffusée dans le tissu corporel et un autre évidement (440) pour le capteur de température ou de chaleur (6),
   - les deux sources de rayonnement (4) ou plus sont reliées à un élément conducteur de lumière (500) qui conduit le rayonnement des au moins deux sources de rayonnement (4, 4') vers la surface du boîtier de capteur (400), et
   - les deux sources de rayonnement (4) ou plus, l'au moins un capteur de rayonnement (5) et le capteur de température ou de chaleur (6) sont disposés sur un circuit commun à l'intérieur du boîtier de capteur (400).

2. Unité de capteur de diagnostic (1) selon la revendication 1, **caractérisée en ce que** l'unité de capteur (1) comprend une unité d'analyse en tant que composant intégré, qui est reliée aux unités de mesure (100, 132, 120, 130) et à l'aide de laquelle tous les signaux de mesure des unités de mesure (100, 132, 120, 130) peuvent être analysés.

3. Unité de capteur de diagnostic (1) selon la revendication 1, **caractérisée en ce qu'**une unité d'analyse (140) séparée est prévue, tous les signaux de mesure des unités de mesure (100, 132, 120, 130) peuvent être transmis, par l'intermédiaire d'une liaison de données, par l'unité de capteur (1) à l'unité d'analyse (140).

4. Unité de capteur de diagnostic (1) selon la revendication 3, **caractérisée en ce que**, grâce à une connexion électrique, par l'intermédiaire de laquelle l'unité de capteur (1) peut être connectée avec un appareil (10) de la technique d'entretien ou de communication ou avec un autre appareil portable ou accessoire.

5. Unité de capteur de diagnostic (1) selon la revendication 3 ou 4, **caractérisée en ce que** le boîtier de capteur (400) est monté, avec toutes les unités de mesure (100, 132, 120, 130), directement sur un câble plat (450) avec un guidage de piste conductrice adapté.

6. Unité de capteur de diagnostic (1) selon la revendication 5, **caractérisée en ce que** le câble plat (450) comprend des renforcements (460).

7. Unité de capteur de diagnostic (1) selon l'une des revendications précédentes, **caractérisée en ce que** le boîtier de capteur (400) présente des dimensions inférieures à 1 cm x 1 cm x 1 cm.

8. Unité de capteur de diagnostic (1) selon l'une des revendications précédentes, **caractérisée en ce qu'**au moins une des électrodes EKG (7) est en même temps l'électrode d'alimentation ou de mesure de l'unité de mesure d'impédance bioélectrique (130).

9. Unité de capteur de diagnostic (1) selon l'une des revendications précédentes, **caractérisée en ce que** l'unité de mesure optique (100) comprend au moins deux capteurs de rayonnement (5) pour la détection du rayonnement diffusé et/ou transmis par le tissu corporel, les capteurs de rayonnement (5) étant disposés à différentes distances de la source de rayonnement (4).

10. Unité de capteur de diagnostic (1) selon l'une des revendications précédentes, **caractérisée en ce que** les deux sources de rayonnement (4, 4') ou plus irradient différentes zones volumiques du tissu corporel examiné.

11. Unité de capteur de diagnostic (1) selon la revendication 10, **caractérisée en ce que** les au moins deux sources de rayonnement (4, 4') ont des caractéristiques de rayonnement spatiales différentes.

12. Unité de capteur de diagnostic (1) selon l'une des revendications précédentes, **caractérisée par** un moyen de détermination de la pression du tissu corporel sur la surface du boîtier de capteur (400).

13. Unité de capteur de diagnostic (1) selon l'une des revendications 4 à 12, **caractérisée en ce que** l'appareil (10) est un appareil mobile, plus particulièrement un notebook, un ordinateur portable, un ordinateur de poche ou un appareil portatif.

SaO2: 97    8
StO2: 70
SvO2: 65
HR:   68
BF:   1.2

BG:   90

**Fig. 1**

**Fig. 2**

**Fig. 3**

**Fig. 4**

**Fig. 5**

**Fig. 6**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2006099988 A1

- WO 2007017266 A2

**In der Beschreibung aufgeführte Nicht-Patentliteratur**

- **MEIR NITZAN ; BORIS KHANOKH.** Infrared Radiometry of Thermally Insulated Skin for the Assessment of Skin Blood Flow. *Optical Engineering,* 1994, vol. 33 (9), 2953-2956